# EUROPEAN PATENT APPLICATION

(11) **EP 4 817 737 A1**
(43) Date of publication of application: **07.10.2026**
(21) Application number: 24899675.3
(22) Date of filing: 26.11.2024
(51) Int. Cl.: B01J 29/70, C07C 211/55

(54) **MODIFIED MOLECULAR SIEVE AND PREPARATION METHOD THEREFOR, CATALYST AND USE THEREOF, AND METHOD FOR SYNTHESIZING DIPHENYLAMINE FROM ANILINE**

(30) Priority: 06.12.2023 CN 202311665591
(71) Applicant: China Petroleum & Chemical Corporation, Beijing 100728 (CN); Sinopec Dalian Research Institute of Petroleum and Petrochemicals Co., Ltd., Lushunkou District Dalian, Liaoning 116045 (CN)
(72) Inventor: WANG, Zhenyu, Dalian, Liaoning 116045 (CN); ZHENG, Bumei, Dalian, Liaoning 116045 (CN); QI, Wenbo, Dalian, Liaoning 116045 (CN); WANG, Libo, Dalian, Liaoning 116045 (CN); ZHAO, Xiangyu, Dalian, Liaoning 116045 (CN)
(74) Representative: karo IP
(86) International application number: PCT/CN2024/134560
(87) International publication number: WO 2025/119035

(57) **Abstract**

A modified molecular sieve and a preparation method therefor, a catalyst and the use thereof, and a method for synthesizing diphenylamine from aniline. The modified molecular sieve comprises a β molecular sieve and an auxiliary component, and the auxiliary component is selected from at least one alkali metal. The indicative constant k of the modified molecular sieve is not less than 100. The catalyst prepared from the modified molecular sieve has a relatively high diphenylamine selectivity and a longer one-way operation period.

## Description

### CROSS-REFERENCE TO RELATED APPLICATIONS

The present application claims the benefit of the China patent application No. "202311665591.X", filed on December 6, 2023, the content of which is specifically and entirely incorporated herein by reference.

### TECHNICAL FIELD

The present invention relates to the technical field of molecular sieve preparation, in particular to a modified molecular sieve and a preparation method therefor, a catalyst and the use thereof, and a method for synthesizing diphenylamine from aniline.

### BACKGROUND ART

Diphenylamine is an important organic chemical raw material with a wide range of uses. It is mainly used in the industries as an antioxidant for synthetic rubber, a stabilizer for explosives, a fuel and pharmaceutical intermediate, azo dyes, a fruit preservative, etc. It can also be used as an analytical reagent for the identification of DNA, the colorimetric determination of nitrates, nitrites, chlorates, and magnesium, and as a redox indicator. At present, the catalysts for the synthesis of diphenylamine from aniline introduced in China and foreign patent technologies are mostly solid acid catalysts, for example, the patent applications US3118944A, US4454348A, US3944613A, CN1114240A have disclosed the technologies of using activated alumina, amorphous silica-alumina and molecular sieves for preparing the catalysts.

In the mid-1990s, the researchers developed a new process for the continuous synthesis of diphenylamine from aniline and the matching special molecular sieve catalyst, whose active component was a Hβ molecular sieve. At present, the continuous synthesis process of diphenylamine from aniline using the Hβ molecular sieve catalyst generally has a conversion rate of 20-25% and a selectivity of 96-97 mol%.

In recent years, researchers in the field have modified the acidity and pore channels of the β molecular sieve catalysts. For example, CN105618106A disclosed a multi-level pore β molecular sieve catalyst rich in a large amount of mesoporous pore volume; the master thesis "Study on Catalyst for Preparation of Diphenylamine by Aniline Condensation" introduced halogens into β molecular sieves to increase acidity; the literature "Effect of Alkali Treatment on the Performance of Hβ Molecular Sieve Catalyzed Aniline Condensation to Prepare Diphenylamine" (Acta Petrolei Sinica, 2017, Vol. 33, No. 1) used alkaline treatment to etch β molecular sieve crystals to increase the mesoporous pore volume, etc. However, there are still problems such as a low selectivity and a short one-way operation period of the catalysts.

### SUMMARY OF THE INVENTION

The present invention aims to overcome the defects in the prior art that the catalysts for synthesizing diphenylamine from aniline have a lower selectivity and a shorter one-way operation period, and provides a modified molecular sieve and a preparation method therefor, a catalyst and the use thereof, and a method for synthesizing diphenylamine from aniline, when the modified molecular sieve is used in a catalyst for synthesizing diphenylamine from aniline, the catalyst has a relatively high catalytic activity, a higher selectivity and a longer one-way operation period.

To achieve the above objects, the first aspect of the present invention provides a modified molecular sieve, the modified molecular sieve comprises a β molecular sieve and an auxiliary component, and the auxiliary component is selected from at least one alkali metal;

Wherein the indicative constant k of the modified molecular sieve is not less than 100.

In the present invention, the indicative constant k is calculated based on formula (1):
$k = Q / \left(1.4909\times {a}^{- 0.558}-c\right)$ formula (1)

Where Q denotes the standard meta-xylene adsorption capacity per 100g of modified molecular sieve, in units of g; a denotes the molar ratio of silica to alumina in the modified molecular sieve; c denotes the molar number of the auxiliary component calculated in terms of oxide per 100g of modified molecular sieve.

The second aspect the present invention provides a preparation method for the modified molecular sieve, the preparation method comprises the following steps:
(1) Preparing an impregnation liquid comprising an organic salt of the auxiliary component and an organic solvent;
   The auxiliary component is selected from at least one alkali metal;
(2) Impregnating a β molecular sieve with the impregnation liquid;
(3) Purging and/or rinsing the product obtained from step (2) with an inactive fluid, such that the organic salt of the auxiliary component carries out the reaction to form a large-molecule product containing the auxiliary component, and the large-molecule product containing the auxiliary component has a molecular diameter greater than the pore diameter of the β molecular sieve to obtain a modified molecular sieve precursor;
(4) Calcining the modified molecular sieve precursor.

The third aspect of the present invention provides another preparation method for the modified molecular sieve, the preparation method comprises the following steps:
(1) Preparing an impregnation liquid comprising an organic salt of the auxiliary component and an organic solvent;
   Wherein the auxiliary component is selected from at least one alkali metal; the organic solvent is a substituted or unsubstituted C6-C10 cyclic hydrocarbon compound; the molecule of said organic salt of the auxiliary component comprises a ring structure, and unsaturated functional groups capable of carrying out the addition reaction with the cyclic hydrocarbon compound;
(2) Impregnating a β molecular sieve with the impregnation liquid;
(3) Purging and/or rinsing the product obtained from step (2) with an inactive fluid at a temperature not lower than 80°C, and then drying to obtain a modified molecular sieve precursor;
(4) Calcining the modified molecular sieve precursor.

The fourth aspect of the present invention provides a modified molecular sieve produced with the preparation method according to the second aspect or the third aspect mentioned above.

The fifth aspect of the present invention provides a catalyst comprising the modified molecular sieve according to the first aspect or the fourth aspect described above and a binder.

The sixth aspect the present invention provides a method for synthesizing diphenylamine from aniline, the method comprises: contacting the aniline with a catalyst under the condensation reaction conditions;

Wherein the catalyst is said catalyst provided in the fifth aspect.

The inventors of the present invention have discovered in research that the reaction of synthesizing diphenylamine from aniline is a typical acid-catalyzed reaction, the acid properties and pore channel structure of the catalyst affect the selectivity of said product and the stability of said catalyst. At present, the industrial catalyst for synthesizing diphenylamine from aniline is a modified β molecular sieve catalyst. The β molecular sieve is a high-silicon molecular sieve with a twelve-membered ring pore structure, which has acidity and pore structure suitable for the reaction of synthesizing diphenylamine from aniline. In the pore structure of β molecular sieve, there are two types of pore space, namely straight-through pores and pore intersections. The pore intersection is formed by that two pore channels penetrate each other at a certain angle and communicate with each other internally. The pore intersection has a wide space, and the acid center is denser than the non-intersection, which can catalyze the cyclization and condensation reaction of aniline molecules to generate macromolecules such as tricyclic linear acridine and bicyclic planar quinoline, and diffuse out from the pores, thereby making it impossible to fundamentally improve the selectivity of diphenylamine. In addition, the pore intersection also becomes an active zone for the generation of carbon deposit precursors.

The indicative constant k of the β molecular sieve modified by the auxiliary agent metal in the prior art is lower than 100, usually within the range of 60-90, a large amount of the auxiliary agent oxide is typically loaded in the straight-through pores, which is not conducive to maintaining the pores unobstructed, affecting the diffusion of reactants and products in the pore channels, and it is difficult to achieve the modification of the acid center at the pore intersections, thereby resulting in a fast decay rate of the aniline conversion rate of the catalyst and a shorter one-way operation period. The time for the first increase in reaction temperature during operation of the industrial catalyst for synthesizing diphenylamine from aniline is generally only 250 hours, and the process of burning carbon and regeneration is required after only 1,500 hours operation of the catalyst, resulting in high operating costs for the production facility and low annual effective operating hours.

Compared with the modified molecular sieves in the prior art, the modified molecular sieve provided by the present invention has an indicative constant k not less than 100, the auxiliary agent components in the modified molecular sieve are almost completely loaded at the pore intersections, can modify the acid center at the pore intersections in a targeted manner without affecting the acid center at the straight-through pores or the smooth flow of the pore channels. The catalyst containing the modified molecular sieve has a relatively high diphenylamine selectivity and a longer one-way operation period.

### DESCRIPTION OF THE PREFERRED EMBODIMENT

The terminals and any value of the ranges disclosed herein are not limited to the precise ranges or values, such ranges or values shall be comprehended as comprising the values adjacent to the ranges or values. As for numerical ranges, the endpoint values of the various ranges, the endpoint values and the individual point values of the various ranges, and the individual point values may be combined with one another to produce one or more new numerical ranges, which should be deemed to have been specifically disclosed herein.

The first aspect the present invention provides a modified molecular sieve, the modified molecular sieve comprises a β molecular sieve and an auxiliary component, and the auxiliary component is selected from at least one alkali metal;

Wherein the indicative constant k of the modified molecular sieve is not less than 100, preferably within the range of 100-550, more preferably within the range of 101-500, for example, it may be 101, 110, 120, 130, 140, 150, 160, 180, 200, 250, 300, 350, 400, 450, 500, and other specific but non-limiting numeric values.

According to the present invention, the modified molecular sieve has an indicative constant k not less than 100, the auxiliary components in the modified molecular sieve are almost completely loaded at the pore intersections, can modify the acid center at the pore intersections in a targeted manner without affecting the acid center at the straight-through pores or the smooth flow of the pore channels. The catalyst containing the modified molecular sieve has a relatively high diphenylamine selectivity and a longer one-way operation period. The indicative constant k of the modified β molecular sieve in the prior art is below 100, a large amount of the auxiliary agent oxide used for modification is typically loaded in the straight-through pores, which is not conducive to maintaining the pores unobstructed, affecting the diffusion of reactants and products in the pore channels, and it is difficult to achieve the modification of the acid center at the pore intersections, thereby resulting in a fast decay rate of the aniline conversion rate of the catalyst and a shorter one-way operation period. The time for the first increase in reaction temperature during operation of the industrial catalyst for synthesizing diphenylamine from aniline is generally only 250 hours, and the process of burning carbon and regeneration is required after only 1,500 hours operation of the catalyst, resulting in high operating costs for the production facility and low annual effective operating hours.

In the present invention, the indicative constant k is calculated based on formula (1):
$k = Q / \left(1.4909\times {a}^{- 0.558}-c\right)$ formula (1)

Wherein Q denotes the standard meta-xylene adsorption capacity per 100g of modified molecular sieve, in units of g; a denotes the molar ratio of silica to alumina in the modified molecular sieve; c denotes the molar number of the auxiliary component calculated in terms of oxide per 100g of modified molecular sieve.

In the present invention, k is a dimensionless numeric value calculated based on formula (1).

The method for measuring the standard meta-xylene adsorption capacity of the modified molecular sieve is as follows: the standard meta-xylene adsorption capacity is measured by using an Intelligent Gravimetric Analyzer (IGA-002). Before measuring the meta-xylene adsorption capacity, the molecular sieve is initially subjected to an impurity removal treatment to eliminate moisture and impurities such as residual templat agents. The impurity removal treatment involves with heating the sample to be tested from room temperature to 300°C at a temperature rise rate of 5°C/minute under a vacuum state, and then cooling to room temperature after the mass of said sample becomes constant, and then measuring the meta-xylene adsorption capacity. At room temperature, meta-xylene vapor is introduced into the sample chamber after impurity removal. After the sample is completely adsorbed and saturated (i.e., the weight is constant), the weight difference is recorded, which is exactly the meta-xylene adsorption capacity of the modified molecular sieve. The ratio of the meta-xylene adsorption capacity to the initial mass of the modified molecular sieve is calculated to obtain the standard meta-xylene adsorption capacity Q per 100g of the modified molecular sieve.

In a further preferred embodiment, the standard meta-xylene adsorption capacity per 100g of modified molecular sieve is within the range of 17-33g, more preferably within the range of 18-31g. In the invention, an use of the meta-xylene as the subject matter can reflect the pore structure of the modified molecular sieve. Under the above preferred circumstance, it indicates that the pore intersections in the modified molecular sieve have a suitable number of acid center and an appropriate pore size, and there are almost no auxiliary components in the straight-through pores, which is conducive to further improving the catalytic selectivity and one-way operation period of the modified molecular sieve catalyst.

In the present invention, the silica-alumina ratio a of the modified molecular sieve refers to the molar ratio of silica and alumina in the modified molecular sieve, and the term "silica-alumina ratio" involved in the invention refers to the molar ratio of silica to alumina, which is obtained by testing with the X-Ray Fluorescence (XRF) characterization method. Preferably, the molar ratio of silica to alumina in the modified molecular sieve is between 20 and 250, more preferably between 25 and 200, for example, it may be 20, 25, 40, 60, 80, 100, 120, 150, and other specific but non-limiting numeric values, or a random value within the range consisting of any two numeric values thereof. Under the aforementioned preferred circumstance, it is beneficial for the modified molecular sieve to retain a suitable active center.

Preferably, the modified molecular sieve does not contain non-framework silica and/or alumina.

Preferably, the β molecular sieve is a Hβ molecular sieve.

According to the invention, c denotes the molar number of the auxiliary component calculated in terms of oxide per 100g of modified molecular sieve, which is obtained by testing with the Inductively Coupled Plasma (ICP) characterization method.

In the present invention, 1.4909 is the fitting coefficient.

According to some preferred embodiments of the invention, the auxiliary component is selected from sodium and/or potassium. In a case of preferably, the auxiliary component exists in the form of an oxide.

According to the present invention, the molar number of the auxiliary component calculated in terms of oxide per 100g of modified molecular sieve is within the range of 0.01-0.12mol, preferably within the range of 0.02-0.1mol.

The existing molecular sieve modification methods (e.g., steam treatment, loading of metal/non-metallic oxides) are a type of general modification methods lacking pertinence, the methods not only modify the pore intersection, but also decorate the straight-through pores. Under the circumstance, there is still a relatively open space and a large number of acid centers at the pore intersections, which can continue to catalyze the occurrence of side reactions, not only affecting the selectivity of the product diphenylamine, but also further forming a carbon deposit precursor with a high carbon-hydrogen ratio, or even forming the carbon deposits. In addition, increasing the loading amount of the modified component is required in the prior art to achieve the purpose of modifying the acid center at the pore intersections, which will affect the diffusion of the reactants and products in the pore channels, increase decay rate of the aniline conversion rate of the catalyst, shorten the one-way operation period. Therefore, the one-way operation period of the industrial catalyst for synthesizing diphenylamine from aniline is relatively short, the time for the first increase in reaction temperature during operation is generally about 250h, and the process of burning carbon and regeneration is required after only 1,500 hours operation of the catalyst, resulting in high operating costs for the production facility and low annual effective operating hours.

In the present invention, by controlling the appropriate loading amount of the modified component and controlling the indicative constant k of the modified molecular sieve within the above range, the acid centers at the pore intersections of the molecular sieve can be regulated while avoiding the influence on the molecular sieve pore channel structure, so that the catalyst made of the modified molecular sieve has a relatively high diphenylamine selectivity and longer one-way operation period.

The second aspect the present invention provides a preparation method for the modified molecular sieve, the preparation method comprises the following steps:
(1) Preparing an impregnation liquid comprising an organic salt of the auxiliary component and an organic solvent;
   The auxiliary component is selected from at least one alkali metal;
(2) Impregnating a β molecular sieve with the impregnation liquid;
(3) Purging and/or rinsing the product obtained from step (2) with an inactive fluid, such that the organic salt of the auxiliary component carries out the reaction to form a large-molecule product containing the auxiliary component, and the large-molecule product containing the auxiliary component has a molecular diameter greater than the pore diameter of the β molecular sieve to obtain a modified molecular sieve precursor;
(4) Calcining the modified molecular sieve precursor.

According to the invention, through step (3), the organic salt of the auxiliary component generates a large-molecule product with a complex spatial structure under the action of the β molecular sieve acid center, the molecular diameter of the large-molecule product is larger than the pore diameter of said molecular sieve. Even under the purging and/or rinsing of an inactive fluid, the substance will continue to remain at the pore intersections of the molecular sieve. The metal oxide formed after further calcination is attached to the pore intersections of the molecular sieve, it plays the roles of modifying the acid center at the pore intersections and adjusting the dimensions of the pore intersections. It is understandable that the large-molecule product cannot be formed in the absence of the catalysis of acid center.

In the present invention, the pore diameter of the β molecular sieve refers to the maximum straight-through pore diameter of the β molecular sieve, when observed with the transmission electron microscopy, the longest diameter of the approximate ellipse of the radial cross section of the pores (perpendicular to the axial direction) is the maximum straight-through pore diameter, and the maximum straight-through pore diameter of the β molecular sieve is generally 0.7 nm. The reaction of an organic salt of the auxiliary component generates a large-molecule product containing the auxiliary component is proved by comparing the composition before and after purging or rinsing. The composition of the liquid phase without purging or rinsing at the same reaction temperature is analyzed by a liquid chromatograph, and the composition of the liquid phase/gas phase after purging or rinsing is analyzed by liquid chromatograph or gas chromatograph. The molecular diameter of the large-molecule product refers to the molecular dynamics diameter, which is calculated by molecular dynamics simulation.

The inactive fluid may be any gas or liquid that does not participate in the reaction.

The present invention has no particular limitation on the type of the reaction, as long as a large-molecule product having a molecular diameter greater than the pore diameter of the β molecular sieve can be obtained. For example, the organic salt of said auxiliary component carries out a self-polymerization reaction to form a large-molecule product containing the auxiliary component, or the organic salt of said auxiliary component carries out an addition reaction with the organic solvent to form a large-molecule product containing the auxiliary component.
method comprises the following steps:
(1) Preparing an impregnation liquid comprising an organic salt of the auxiliary component and an organic solvent;
   Wherein the auxiliary component is selected from at least one alkali metal; the organic solvent is a substituted or unsubstituted C6-C10 cyclic hydrocarbon compound;
   The molecule of said organic salt of the auxiliary component comprises a ring structure, and unsaturated functional groups capable of carrying out the addition reaction with the cyclic hydrocarbon compound;
(2) Impregnating a β molecular sieve with the impregnation liquid;
(3) Purging and/or rinsing the product obtained from step (2) with an inactive fluid at a temperature not lower than 80°C, and then drying to obtain a modified molecular sieve precursor;
(4) Calcining the modified molecular sieve precursor.

The existing molecular sieve modification method (e.g., steam treatment, loading of metal/non-metallic oxides) are a type of general modification means lacking pertinence, the methods not only modify the pore intersection, but also decorate the straight-through pore channels. In the prior art, if the purpose of modifying the acid center at the pore intersections is to be achieved, a high loading amount of the modifier is required, which is not conducive to the diffusion of reactants and products in the pores, thereby increasing the attenuation rate of the aniline conversion rate of the catalyst and shortening the one-way operation period.

In the preparation method for the modified molecular sieve provided by the present invention, the organic salt of the auxiliary component is initially dissolved in the cyclic hydrocarbon compound to prepare an impregnation liquid, the β molecular sieve pores are then filled with the impregnation liquid through an impregnation treatment, so that the organic salt of the auxiliary component and the cyclic hydrocarbon compound diffuse into the molecular sieve pores; further purging and/or rinsing with an inactive fluid not less than 80°C, the unsaturated functional groups in the molecules of the organic salt of the auxiliary component are subjected to the in-situ addition reaction with the cyclic hydrocarbon compound under the high temperature to generate a large-molecule salt product with a multi-ring structure. Since the molecular size of the reaction product is larger than the diameter of straight-through pores of the molecular sieve, even if there is an acid center required for the reaction, it cannot be generated in the straight-through pores, and can only be generated at the pore intersections, where there is sufficient space and an acid center. After further calcination, the formed metal oxide is attached to the pore intersections of the molecular sieve, which plays a role of modifying the acid center at the pore intersections and adjusting the size of the pore intersections.

The modified molecular sieve produced with the preparation method provided by the present invention has a higher diphenylamine selectivity and a longer one-way operation period when used in catalysts. The reason may be that the aforementioned preparation method can modify the acid centers at the pore intersection of the β molecular sieve without affecting the acid centers at the straight-through pores or the smooth flow of the pore channels.

According to some preferred embodiments of the invention, the ring structure is at least one selected from the group consisting of a saturated or unsaturated five-membered ring and a six-membered ring. The ring structure may be a carbon ring consisting entirely of carbon atoms, or may be a heterocyclic ring containing heteroatoms such as S, N, O. Preferably, the saturated or unsaturated five-membered ring and six-membered ring are all composed of carbon atoms.

According to the present invention, the β molecular sieve is preferably a Hβ molecular sieve. The present invention is not particularly limited with respect to the source of the β molecular sieve, it is commercially available or may be prepared with any method known in the art. Preferably, the molar ratio of silica to alumina in the modified molecular sieve is between 20 and 250, more preferably between 25 and 200.

Preferably, the molecule of the organic salt of the auxiliary component comprises at least one unsaturated six-membered ring, more preferably a benzene ring. In the above preferred case, it is conducive to the generation of large-molecule salt products with a multi-ring structure.

According to the present invention, the molecule of the organic salt of the auxiliary component also contains unsaturated functional groups capable of performing an addition reaction with the cyclic hydrocarbon compound. The present invention has no particular limitation on the type of the unsaturated functional groups, as long as the unsaturated functional groups can perform an addition reaction with the cyclic hydrocarbon compound. Preferably, the unsaturated functional groups are selected from carbon-carbon double bond and/or carbon-carbon triple bond. The present invention has no particular limitation on the position of the unsaturated functional groups in the molecule, the unsaturated functional groups may be disposed on the ring of the ring structure, or in a substituent of the ring structure.

In the invention, the addition reaction is carried out under certain temperature conditions and catalysis by the acid center of β molecular sieve.

In a further preferred embodiment, the organic salt of the auxiliary component has a molecular weight not more than 250g/mol, more preferably within the range of 50-220g/mol. Controlling the molecular weight of the organic salt of the auxiliary component within the above preferred range is conducive to the generation of large-molecule salt products with a multi-ring structure of suitable molecular size at the pore intersection of the molecular sieve.

According to a particularly preferred embodiment of the present invention, the organic salt of the auxiliary component has a structure represented by formula (i); 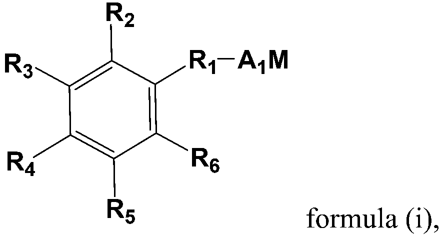 wherein R₁ is C2-C4 alkenylene or alkynylene, for example, it may be at least one of ethenylene, ethynylene, propenylene, propynylene, butenylene, and butynylene. The position of the carbon-carbon double bond or carbon-carbon triple bond can be located in the middle of the group or at both ends of the group, and the present invention has no particular limitation. The alkenylene or alkynylene can be a straight chain or branched chain group, and the present invention has no particular limitation thereto either.

In formula (i), R₂, R₃, R₄, R₅, R₆ are each independently at least one selected from the group consisting of hydrogen atom and C1-C3 hydrocarbyl, for example, they may be at least one of a hydrogen atom, methyl, and ethyl, more preferably a hydrogen atom.

In formula (i), A₁ is selected from -COO- or -SO₃-, preferably -COO-.

In formula (i), M refers to an auxiliary agent metal ion, which is selected from at least one alkali metal ion, preferably K⁺ or Na⁺.

The above preferred implementation is beneficial for the auxiliary component to modify the acid centers at the pore intersections of the molecular sieve, and will not affect the smooth flow of the pore channels, so that the catalyst made of the modified molecular sieve has a higher diphenylamine selectivity and a longer one-way operation period.

In the invention, on one hand, the cyclic hydrocarbon compound acts as a solvent in the impregnation liquid, such that the organic salt of the auxiliary component is evenly dispersed in the cyclic hydrocarbon compound; on the other hand, the cyclic hydrocarbon compound and the organic salt of the auxiliary component can perform an addition reaction at the pore intersections of the molecular sieve under the conditions of purging or rinsing with an inactive fluid and a temperature not lower than 80°C to generate a large-molecule organic salt.

The present invention has a wide selection range for the cyclic hydrocarbon compound, preferably substituted or unsubstituted C6-C10 cyclic hydrocarbon compound, the substituent in the cyclic hydrocarbon compound may be, for example, C1-C3 alkyl; further preferably, the cyclic hydrocarbon compound is at least one selected from the group consisting of benzene, cyclohexane, toluene, methylcyclopentane and cyclohexene.

According to a particularly preferred embodiment of the present invention, the organic salt of the auxiliary component is a cinnamate of the auxiliary component, and the cyclic hydrocarbon compound is at least one selected from the group consisting of benzene, cyclohexane, toluene, methylcyclopentane and cyclohexene, more preferably benzene. An use of the preferred embodiment is helpful for that the two components form a uniform liquid phase and generate a large-molecule salt product with a multi-ring structure at the pore intersection of the molecular sieve.

According to some preferred embodiments of the present invention, based on the total amount of the impregnation liquid, the organic salt of the auxiliary component is contained in an amount of 0.1-10wt%, more preferably 0.5-9wt%; the cyclic hydrocarbon compound is contained in an amount of 90-99.9wt%, preferably 90-99.5wt%. The preferred embodiments are conducive to the formation of a uniform liquid phase and the reaction to generate an appropriate amount of large-molecule salt products with a multi-ring structure to fill the pore intersections of the molecular sieve.

According to some preferred embodiments of the invention, the dosage of said impregnation liquid is 4-10mL, more preferably 5-8mL, relative to 1g of said Hβ molecular sieve.

The present invention does not particularly limit the impregnation temperature, those skilled in the art can adjust the impregnation temperature according to actual needs. Preferably, the impregnation can be carried out at room temperature, the impregnation time is preferably within the range of 1-10h, more preferably within the range of 1-5h. Under the preferred circumstance, it can be ensured that the impregnation liquid fills the pores of the Hβ molecular sieve, so that the organic salt of auxiliary component and the cyclic hydrocarbon compound diffuse into the pores of the molecular sieve.

In the present invention, the inactive fluid may be any gas or liquid that does not participate in the reaction, for example, it may be at least one selected from the group consisting of nitrogen, argon, helium and neon. From the perspective of reducing the preparation cost, nitrogen is preferred.

According to the invention, the high-temperature purging and/or rinsing process not only provides the required temperature for the addition reaction, but also can vaporize the excess small molecules in the pores and take them out of the molecular sieve, while the large-molecule products generated by the reaction cannot be taken out of the pores and continue to remain at the pore intersections due to the molecular size limitation. Preferably, the content of said cyclic hydrocarbon compound in the inactive fluid at an outlet is not higher than 1 µg/L at the end of purging and/or rinsing process.

Preferably, the purging and/or rinsing conditions comprise: the inactive fluid temperature is within the range of 80-300°C, more preferably within the range of 85-280°C, the hourly volumetric space velocity is within the range of 100-3,000 h⁻¹, more preferably within the range of 200-2,000 h⁻¹, the purging and/or rinsing time is within the range of 2-24h, more preferably within the range of 3-20h. An use of the preferred embodiment is conducive to the formation of large-molecule products to modify the acid centers at the pore intersections of the Hβ molecular sieve, and can remove excess small molecules in the pore channels to avoid an influence on the smooth flow of the pore channels, such that the catalyst made of the modified molecular sieve has a higher diphenylamine selectivity and a longer one-way operation period.

In the present invention, the remaining and unreacted organic salts of the auxiliary components and cyclic hydrocarbon compounds in the product can be further removed by an optional washing step. The present invention does not specifically limit the operation mode and conditions of the washing process, and those skilled in the art can make choices according to actual requirements.

According to some preferred embodiments of the present invention, the detergent adopted in the washing process may be water, preferably, the liquid-to-solid ratio of water to the product obtained from the purging process is 4-10 mL/g, more preferably 5-8 mL/g; the washing temperature may be 20-80°C, more preferably 20-70°C, and from the perspective of energy saving, the washing may be performed at room temperature. The present invention does not particularly limit the number of washing operation, and can be adjusted according to the actual washing condition. Preferably, the number of washing operation is 1-10 times, preferably 2-6 times.

The present invention has no particular limitation on the drying conditions. Preferably, the drying temperature is 60-150°C, more preferably 80-120°C, and the drying time is 2-24 h, more preferably 3-20 h.

In the present invention, the calcining in step (4) is performed so that the carbon and hydrogen components in the large-molecule product carried in the modified molecular sieve precursor are fully oxidized and combusted to become carbon dioxide and water vapor and escape from the molecular sieve, and the remaining metal oxides continue to adhere to the pore intersections of the molecular sieve, playing the roles of modifying the acid center at the pore intersections and adjusting the size of the pore intersection. Preferably, the calcining conditions comprise: a temperature within the range of 300-800°C, more preferably within the range of 400-700°C, and a calcining time within the range of 2-24h, more preferably within the range of 3-8h.

The present invention further provides a method for preparing a molecular sieve, the method comprises the following steps:
(1) Preparing an impregnation liquid comprising an organic salt of the auxiliary component and a cyclic hydrocarbon compound;
   Wherein the molecule of the organic salt of the auxiliary component comprises a ring structure and contains a carbon-carbon double bond and/or a carbon-carbon triple bond;
   The auxiliary component is selected from at least one alkali metal; the organic solvent is a substituted or unsubstituted C6-C10 cyclic hydrocarbon compound;
(2) Impregnating a β molecular sieve with the impregnation liquid;
(3) Purging the product obtained from step (2) with an inactive gas with a temperature not lower than 80°C, and then optionally washing and drying to obtain a modified molecular sieve precursor;
(4) Calcining the modified molecular sieve precursor.

Particularly preferably, the present invention provides a preferred method for preparing a modified molecular sieve, the method comprises the following steps:
(1) Preparing an impregnation liquid comprising an organic salt of the auxiliary component and a cyclic hydrocarbon compound;
   Wherein the organic salt of the auxiliary component is cinnamate, the auxiliary component is selected from at least one alkali metal; the cyclic hydrocarbon compound is at least one selected from the group consisting of benzene, cyclohexane, toluene, methylcyclopentane and cyclohexene;
(2) Impregnating a β molecular sieve with the impregnation liquid;
(3) Purging the product obtained from step (2) with an inactive gas with a temperature not lower than 80°C, and then optionally washing and drying to obtain a modified molecular sieve precursor;
(4) Calcining the modified molecular sieve precursor.

According to the present invention, the cinnamate and the above-mentioned cyclic hydrocarbon compound can perform an addition reaction under the catalytic action of the acid center of the β molecular sieve at a certain temperature to obtain a large-molecule compound containing a multi-ring. The metal oxide obtained after the calcining process is attached to the pore intersections of the molecular sieve, playing the roles of modifying the acid center at the pore intersections of the channels and adjusting the size of the pore intersections.

The fourth aspect of the present invention provides a modified molecular sieve produced with the aforementioned preparation method

The fifth aspect of the present invention provides a catalyst comprising the modified molecular sieve according to the first aspect or the fourth aspect described above and a binder.

The present invention has a wide range of choices for the binder, it can be any conventional choice in the art, for example, the binder may be at least one selected from the group consisting of alumina, bentonite, spinel and silica, preferably alumina.

According to some preferred embodiments of the invention, the modified molecular sieve is contained in an amount of 55-85wt%, more preferably 58-83wt%, and the binder is contained in an amount of 15-45wt%, more preferably 17-42wt%, based on the total amount of the catalyst.

According to the invention, preferably, the catalyst further comprises a second auxiliary component, which can be any metal component and/or non-metal component that is beneficial to improving the catalytic activity of the catalyst in synthesizing diphenylamine from aniline.

To further improve the selectivity and the one-way operation period of the catalyst, preferably, the metal component is at least one metal element selected from group IA, group IIA, group IIIB, group IVB, group VIII, group IB, group IIB, for example, it can be at least one of Li, Na, K, Mg, Ca, Ti, Zr, La, Ce, Pr, Fe, Co, Ni, Cu and Zn, preferably at least one of Li, La, Ce, Mg, Ca, Ba, Cu, Zn, Zr, Fe, and more preferably at least one of La, Ce and Mg.

Preferably, the non-metallic component is at least one selected from group IIIA nonmetal element, group IVA nonmetal element and group VA nonmetallic element, more preferably at least one of Si, P, B and C.

Preferably, the content of said second auxiliary component calculated in terms of oxide is within the range of 0.1-5wt%, more preferably within the range of 1-4wt%, based on the total amount of the catalyst. Under the preferred embodiment, it is beneficial to further adjust acidity of the catalyst, thereby further improving the catalyst activity.

According to the invention, preferably, the catalyst has a specific surface area within the range of 250-650 m²/g, more preferably within the range of 300-600 m²/g.

According to the present invention, preferably, the catalyst has a specific pore volume within the range of 0.2-0.55 mL/g, more preferably within the range of 0.25-0.5 mL/g.

In the present invention, the specific surface area and specific pore volume of the catalyst are measured by the nitrogen physical adsorption method.

The invention has no particular requirements for the shape of the catalyst, which may be in the form of a strip or a sphere. When in the form of a strip, its cross-section may be cylindrical, three-leaf clover or four-leaf clover, etc. The strip diameter is preferably within the range of 0.5-3 mm (according to the Enterprise Standard Q/SH 361 933 of Sinopec Catalyst Co., Ltd.), preferably within the range of 1-2mm; when the cross-section of the strip is in the form of a sphere, its diameter is preferably within the range of 0.5-5mm, more preferably within the range of 1-3mm.

The present invention has no particular limitation on the preparation method of the catalyst, and the catalyst can be prepared by any conventional method in the art. For example, initially molding the modified molecular sieve and the binder, and then loading the second auxiliary component. The present invention does not impose specific limitation on the method of loading the second auxiliary component, and the second auxiliary component can be loaded by a conventional loading method, such as an impregnation method, which is well-known among those skilled in the art.

Another aspect of the present invention provides a method for preparing a catalyst, the method comprises the following steps:
(1) Preparing an impregnation liquid comprising an organic salt of the auxiliary component and a cyclic hydrocarbon compound;
   Wherein the molecule of the organic salt of the auxiliary component comprises a ring structure, and an unsaturated functional group capable of performing an addition reaction with the cyclic hydrocarbon compound;
   The auxiliary component is selected from at least one alkali metal; the cyclic hydrocarbon compound is a substituted or unsubstituted C6-C10 cyclic hydrocarbon compound;
(2) Impregnating a β molecular sieve with the impregnation liquid;
(3) Purging and/or rinsing the product obtained from step (2) with an inactive fluid with a temperature not lower than 80°C, and then optionally washing and drying to obtain a modified molecular sieve precursor;
(4) Calcining the modified molecular sieve precursor to obtain a modified molecular sieve;
(5) Mixing and molding the modified molecular sieve, a binder and/or a binder precursor, an optional extrusion aid, and an optional peptizing agent, and then performing a first drying and a first calcination to obtain a catalyst precursor;
(6) Impregnating the catalyst precursor with a solution containing a soluble compound of a second auxiliary component, and then performing a second drying and a second calcination.

The specific operating conditions of step (1) to step (4) are the same as those defined in the second aspect and will not be repeatedly described herein.

In the present invention, the binder precursor refers to any substance that can be calcined to obtain the binder. For example, the precursor of aluminum oxide may be pseudo-boehmite, which is well known among those skilled in the art.

The present invention has no particular limitation on the extrusion aid and the peptizing agent, both can be conventional choices in the art. The extrusion aid can be, for example, sesbania powder, and the peptizing agent may be dilute nitric acid and/or citric acid. The concentration of said dilute nitric acid may be generally within the range of 3-15wt%.

According to some preferred embodiments of the invention, the mass ratio of the modified molecular sieve, the binder and/or the binder precursor, the extrusion aid and the peptizing agent is (60-85):(15-40):(3-20):(5-80), preferably (70-80):(20-30):(10-15):(20-50), wherein the weights of the modified molecular sieve, the binder and/or the binder precursor are calculated on a dry basis.

In the present invention, the dry basis weight of the modified molecular sieve refers to the weight of the modified molecular sieve after calcining at 600°C to burn off the adsorbed water and crystal water.

The dry basis weight of the binder and/or binder precursor refers to the weight of the binder and/or binder precursor after calcining at 600°C to burn off the adsorbed water and crystal water.

The present invention has no particular limitation on the molding mode, those skilled in the art can select the molding mode according to actual needs, for example, it can be extrusion molding, tablet molding, ball rolling molding, etc.

According to some preferred embodiments of the invention, the conditions for the first drying and the second drying each independently comprise: a temperature of 60-150°C, more preferably 80-120°C, and a time of 2-24 h, more preferably 5-20 h.

According to some preferred embodiments of the present invention, before performing the second drying, the product obtained by the impregnation is naturally dried in the shade, for example, the product is placed at room temperature for drying for 10-48 hours.

Preferably, the conditions of the first calcination and the second calcination each independently comprise: a temperature of 300-800°C, more preferably 400-700°C, and a calcination time of 2-24 h, more preferably 3-8 h.

The present invention has no particular requirements for the specific operation and conditions of the impregnation in step (5), as long as meeting the requirement of the loading amount of the second auxiliary component. Preferably, the content of said second auxiliary component calculated in terms of oxide is within the range of 0.1-5wt%, more preferably within the range of 1-4wt%, based on the total amount of the catalyst.

The soluble compound of the second auxiliary component may be a conventional inorganic salt and/or organic salt containing the second auxiliary component, and the solvent in the impregnation liquid is preferably water.

The sixth aspect of the present invention provides an use of the aforementioned modified molecular sieve or the aforementioned catalyst in an aniline condensation reaction.

The seventh aspect of the present invention provides a method for synthesizing diphenylamine from aniline, the method comprises: contacting the aniline with a catalyst under the condensation reaction conditions; wherein the catalyst is the catalyst according to the fifth aspect.

According to the present invention, preferably, the condensation reaction conditions comprise: a reaction pressure within the range of 0.1-4MPa, more preferably within the range of 0.15-3.5MPa; a reaction temperature within the range of 280-360°C, more preferably within the range of 290-350°C; an aniline hourly volumetric space velocity within the range of 0.1-0.3h⁻¹, more preferably within the range of 0.15-0.25h⁻¹. Unless otherwise specified, the pressure involved in the invention refers to the gauge pressure.

The present invention will be described in detail below with reference to examples.

In the following examples, unless otherwise specified, the raw materials in use were all commercially available, and the weights of the modified molecular sieve and the alumina precursor in the material dosages were weights on a dry basis.

The following preparation examples were used to illustrate the preparation of modified molecular sieves.

### Preparation Example 1

(1) An appropriate amount of potassium cinnamate was dissolved in benzene, uniformly stirred and fully dissolved, wherein the percentage content by mass of potassium cinnamate was 8.5 wt%; 200g of a Hβ molecular sieve (the molar ratio of silica to alumina was 40) was impregnated with the solution at a liquid-solid ratio of 5 mL/g, after impregnation for 2 hours, the molecular sieve was filtered;
(2) The filtered molecular sieve was purged with hot nitrogen gas at 110°C with a hourly volumetric space velocity of 800 h⁻¹ for 4 hours until the benzene content in the nitrogen gas at an outlet was no higher than 1 µg/L;
   The molecular sieve was naturally cooled to room temperature after completion of the purging process;
(3) The molecular sieve obtained from step (2) was washed with deionized water at a liquid-to-solid ratio of 6 mL/g, and the washed molecular sieve was subjected to drying at 110°C for 5 hours and calcination at 550°C for 5 hours, a modified Hβ molecular sieve A1 was obtained; the composition and physicochemical properties of the modified molecular sieve were shown in Table 1.

### Preparation Example 2

(1) An appropriate amount of potassium cinnamate was dissolved in benzene, uniformly stirred and fully dissolved, wherein the percentage content by mass of potassium cinnamate was 9 wt%; 200g of a Hβ molecular sieve (the molar ratio of silica to alumina was 25) was impregnated with the solution at a liquid-solid ratio of 7 mL/g, after impregnation for 4 hours, the molecular sieve was filtered;
(2) The filtered molecular sieve was purged with hot nitrogen gas at 120°C with a hourly volumetric space velocity of 1,000 h⁻¹ for 4 hours, the molecular sieve was naturally cooled to room temperature after completion of the purging process;
(3) The molecular sieve obtained from step (2) was washed with deionized water at a liquid-to-solid ratio of 5 mL/g, and the washed molecular sieve was subjected to drying at 110°C for 4 hours and calcination at 500°C for 5 hours, a modified Hβ molecular sieve A2 was obtained; the composition and physicochemical properties of the modified molecular sieve were shown in Table 1.

### Preparation Example 3

(1) An appropriate amount of potassium cinnamate was dissolved in benzene, uniformly stirred and fully dissolved, wherein the percentage content by mass of potassium cinnamate was 8 wt%; 200g of a Hβ molecular sieve (the molar ratio of silica to alumina was 80) was impregnated with the solution at a liquid-solid ratio of 7 mL/g, after impregnation for 3 hours, the molecular sieve was filtered;
(2) The filtered molecular sieve was purged with hot nitrogen gas at 120°C with a hourly volumetric space velocity of 1,100 h⁻¹ for 3 hours, the molecular sieve was naturally cooled to room temperature after completion of the purging process;
(3) The molecular sieve obtained from step (2) was washed with deionized water at a liquid-to-solid ratio of 8 mL/g, and the washed molecular sieve was subjected to drying at 110°C for 3 hours and calcination at 550°C for 3 hours, a modified Hβ molecular sieve A3 was obtained; the composition and physicochemical properties of the modified molecular sieve were shown in Table 1.

### Preparation Example 4

(1) An appropriate amount of potassium cinnamate was dissolved in benzene, uniformly stirred and fully dissolved, wherein the percentage content by mass of potassium cinnamate was 6 wt%; 200g of a Hβ molecular sieve (the molar ratio of silica to alumina was 150) was impregnated with the solution at a liquid-solid ratio of 5 mL/g, after impregnation for 4 hours, the molecular sieve was filtered;
(2) The filtered molecular sieve was purged with hot nitrogen gas at 110°C with a hourly volumetric space velocity of 900 h⁻¹ for 5 hours, the molecular sieve was naturally cooled to room temperature after completion of the purging process;
(3) The molecular sieve obtained from step (2) was washed with deionized water at a liquid-to-solid ratio of 5 mL/g, and the washed molecular sieve was subjected to drying at 120°C for 6 hours and calcination at 540°C for 4 hours, a modified Hβ molecular sieve A4 was obtained; the composition and physicochemical properties of the modified molecular sieve were shown in Table 1.

### Preparation Example 5

The modified molecular sieve was prepared according to the method of Preparation Example 1, except that in step (1), the percentage content by mass of potassium cinnamate was 2 wt%. The obtained modified molecular sieve was denoted as A5, the composition and physicochemical properties of the modified molecular sieve were shown in Table 1.

### Preparation Example 6

The modified molecular sieve was prepared according to the method of Preparation Example 1, except that in step (2), the filtered molecular sieve was purged with hot nitrogen gas at 80°C with a hourly volumetric space velocity of 150 h⁻¹ for 3 hours. The obtained modified molecular sieve was denoted as A6, the composition and physicochemical properties of the modified molecular sieve were shown in Table 1.

### Comparative Preparation Example 1

An aqueous solution of potassium nitrate was used as the impregnation liquid, a Hβ molecular sieve (the molar ratio of silica to alumina was 40) was impregnated with an equivalent-volume impregnation method, after impregnation for 2 hours, the molecular sieve was filtered, it was subjected to drying at 110°C for 5 hours and calcination at 550°C for 5 hours, the obtained modified molecular sieve was denoted as DA1, the composition and physicochemical properties of the modified molecular sieve were shown in Table 1.

### Comparative Preparation Example 2

An aqueous solution of potassium cinnamate was used as the impregnation liquid, a Hβ molecular sieve (the molar ratio of silica to alumina was 25) was impregnated with an equivalent-volume impregnation method, after impregnation for 2 hours, the molecular sieve was filtered, it was subjected to drying at 110°C for 5 hours and calcination at 550°C for 5 hours, the obtained modified molecular sieve was denoted as DA2, the composition and physicochemical properties of the modified molecular sieve were shown in Table 1.

### Comparative Preparation Example 3

An aqueous solution of potassium nitrate was used as the impregnation liquid, a Hβ molecular sieve (the molar ratio of silica to alumina was 80) was impregnated with an equivalent-volume impregnation method, after impregnation for 3 hours, the molecular sieve was filtered, it was subjected to drying at 120°C for 3 hours and calcination at 560°C for 5 hours, the obtained modified molecular sieve was denoted as DA3, the composition and physicochemical properties of the modified molecular sieve were shown in Table 1.

### Comparative Preparation Example 4

The unmodified Hβ molecular sieve (the molar ratio of silica to alumina was 40) was denoted as DA4, the composition and physicochemical properties of the molecular sieve were shown in Table 1.

**Table 1**

| Modified molecular sieve | Silica/alumina molar ratio | Molar amount of auxiliary component calculated in terms of oxide mol/100g | Meta-xylene adsorption capacity g/100g | Indicative constant, k |
|---|---|---|---|---|
| A1 | 40 | 0.02 | 23.23 | 137 |
| A2 | 25 | 0.03 | 24.45 | 113 |
| A3 | 80 | 0.04 | 22.80 | 255 |
| A4 | 150 | 0.03 | 21.67 | 361 |
| A5 | 40 | 0.01 | 23.56 | 130 |
| A6 | 40 | 0.02 | 17.38 | 102 |
| DA1 | 40 | 0.02 | 12.92 | 76 |
| DA2 | 25 | 0.03 | 13.22 | 61 |
| DA3 | 80 | 0.04 | 7.48 | 84 |
| DA4 | 40 | 0 | 25.89 | 138 |

The following examples served to illustrate the preparation of the catalyst.

### Example 1

(1) The modified molecular sieve A1, alumina, sesbania powder and 10wt% nitric acid solution were fully mixed and kneaded in a mass ratio of 70:30:10:40, wherein the weight of the modified molecular sieve and alumina precursor were calculated on a dry basis, and then extruded and molded into a three-leaf clover strip with a cross-sectional diameter of 2 mm. The mixture was subjected to drying at 110°C for 5 hours and calcination at 550°C for 5 hours, a catalyst precursor was obtained;
(2) The catalyst precursor obtained in step (1) was loaded with a Lanthanum (La) oxide by an equivalent-volume impregnation method, and then subjected to drying at 110°C for 5 hours and calcination at 550°C for 5 hours to obtain a finished product catalyst, which was denoted as CAT-1. The composition and physicochemical properties of the catalyst were shown in Table 2.

### Example 2

(1) The modified molecular sieve A2, alumina, sesbania powder and 12wt% nitric acid solution were fully mixed and kneaded in a mass ratio of 77:23:11:38, wherein the weight of the modified molecular sieve and alumina precursor were calculated on a dry basis, and then extruded and molded into a cylindrical shape with a cross-sectional diameter of 1.8 mm. The mixture was subjected to drying at 120°C for 4 hours and calcination at 510°C for 3 hours, a catalyst precursor was obtained;
(2) The catalyst precursor obtained in step (1) was loaded with a Cerium (Ce) oxide by an equivalent-volume impregnation method, and then subjected to drying at 120°C for 5 hours and calcination at 500°C for 4 hours to obtain a finished product catalyst, which was denoted as CAT-2. The composition and physicochemical properties of the catalyst were shown in Table 2.

### Example 3

(1) The modified molecular sieve A3, alumina, sesbania powder and 15wt% nitric acid solution were fully mixed and kneaded in a mass ratio of 72:28:13:35, wherein the weight of the modified molecular sieve and alumina precursor were calculated on a dry basis, and then extruded and molded into a cylindrical shape with a cross-sectional diameter of 1.5 mm. The mixture was subjected to drying at 120°C for 5 hours and calcination at 550°C for 3 hours, a catalyst precursor was obtained;
(2) The catalyst precursor obtained in step (1) was loaded with a Magnesium (Mg) oxide by an equivalent-volume impregnation method, and then subjected to drying at 120°C for 5 hours and calcination at 550°C for 4 hours to obtain a finished product catalyst, which was denoted as CAT-3. The composition and physicochemical properties of the catalyst were shown in Table 2.

### Example 4

(1) The modified molecular sieve A4, alumina, sesbania powder and 10wt% nitric acid solution were fully mixed and kneaded in a mass ratio of 60:40:15:40, wherein the weight of the modified molecular sieve and alumina precursor were calculated on a dry basis, and then extruded and molded into a three-leaf clover strip with a cross-sectional diameter of 2.2 mm. The mixture was subjected to drying at 110°C for 6 hours and calcination at 550°C for 4 hours, a catalyst precursor was obtained;
(2) The catalyst precursor obtained in step (1) was loaded with a Si oxide by an equivalent-volume impregnation method, and then subjected to drying at 110°C for 5 hours and calcination at 550°C for 5 hours to obtain a finished product catalyst, which was denoted as CAT-4. The composition and physicochemical properties of the catalyst were shown in Table 2.

### Example 5

The catalyst was prepared according to the method of Example 1, except that the modified molecular sieve A1 was replaced with the modified molecular sieve A5. The finished product catalyst was denoted as CAT-5, the composition and physicochemical properties of the catalyst were shown in Table 2.

### Example 6

The catalyst was prepared according to the method of Example 1, except that the modified molecular sieve A1 was replaced with the modified molecular sieve A6. The finished product catalyst was denoted as CAT-6, the composition and physicochemical properties of the catalyst were shown in Table 2.

### Example 7

The catalyst was prepared according to the method of Example 1, except that step (2) was not carried out, and the catalyst precursor obtained in step (1) was taken directly as the finished product catalyst, which was denoted as CAT-7.

### Comparative Example 1

The catalyst was prepared according to the method of Example 1, except that the modified molecular sieve A1 was replaced with the modified molecular sieve DA1. The finished product catalyst was denoted as DCAT-1, the composition and physicochemical properties of the catalyst were shown in Table 2.

### Comparative Example 2

The catalyst was prepared according to the method of Example 2, except that the modified molecular sieve A2 was replaced with the modified molecular sieve DA2. The finished product catalyst was denoted as DCAT-2, the composition and physicochemical properties of the catalyst were shown in Table 2.

### Comparative Example 3

The catalyst was prepared according to the method of Example 3, except that the modified molecular sieve A3 was replaced with the modified molecular sieve DA3. The finished product catalyst was denoted as DCAT-3, the composition and physicochemical properties of the catalyst were shown in Table 2.

### Comparative Example 4

The catalyst was prepared according to the method of Example 1, except that the modified molecular sieve A1 was replaced with the modified molecular sieve DA4. The finished product catalyst was denoted as DCAT-4, the composition and physicochemical properties of the catalyst were shown in Table 2.

### Comparative Example 5

(1) The modified molecular sieve DA1, alumina, sesbania powder and 10wt% nitric acid solution were fully mixed and kneaded in a mass ratio of 70:30:10:40, wherein the weight of the modified molecular sieve and alumina precursor were calculated on a dry basis, and then extruded and molded, and further subjected to drying at 110°C for 5 hours and calcination at 550°C for 5 hours, a catalyst precursor was obtained;
(2) The catalyst precursor obtained in step (1) was contacted with an aqueous solution of molybdenum nitrate, and NaBH₄ was added, the mixture was subjected to backflow reduction at 100°C for 8 hours, and then subjected to filtering and vacuum drying to obtain a spherical catalyst with a particle diameter of 3 mm, which was denoted as DCAT-5.

The composition and physicochemical properties of the catalyst were shown in Table 2. The standard meta-xylene adsorption capacity per 100g of the catalyst DCAT-5 was 10.65 g, and the calculated indicative constant k was 36.

**Table 2**

| Catalyst | Modified molecular sieve, content wt% | Binder, content wt% | Second auxiliary component, content wt% | Specific surface area, m²/g | Specific pore volume mL/g |
|---|---|---|---|---|---|
| CAT-1 | A1, 68.3wt% | Alumina, 29.2wt% | La₂O₃, 2.5wt% | 512.35 | 0.47 |
| CAT-2 | A2, 74.7wt% | Alumina, 22.3wt% | CeO₂, 3wt% | 508.24 | 0.47 |
| CAT-3 | A3, 70.6wt% | Alumina, 27.4wt% | MgO, 2wt% | 499.86 | 0.46 |
| CAT-4 | A4, 58.5wt% | Alumina, 39wt% | SiO₂, 2.5wt% | 483.67 | 0.46 |
| CAT-5 | A5, 68.3wt% | Alumina, 29.2wt% | La₂O₃, 2.5wt% | 525.31 | 0.47 |
| CAT-6 | A6, 68.3wt% | Alumina, 29.2wt% | La₂O₃, 2.5wt% | 519.55 | 0.47 |
| CAT-7 | A1, 70wt% | Alumina, 30wt% | / | 535.61 | 0.46 |
| DCAT-1 | DA1, 68.3wt% | Alumina, 29.2wt% | La₂O₃, 2.5wt% | 519.58 | 0.47 |
| DCAT-2 | DA2, 73.5wt% | Alumina, 23.5wt% | CeO₂, 3wt% | 501.26 | 0.46 |
| DCAT-3 | DA3, 71.8wt% | Alumina, 26.2wt% | SiO₂, 2wt% | 513.67 | 0.47 |
| DCAT-4 | DA4, 68.3wt% | Alumina, 29.2wt% | La₂O₃, 2.5wt% | 542.36 | 0.47 |
| DCAT-5 | DA1, 68.3wt% | Alumina, 29.2wt% | MoO, 2.5wt% | 502.38 | 0.46 |

### Test Example

The catalysts of the above-mentioned examples and comparative examples were used in a small-size evaluation device to carry out an evaluation experiment of synthesizing diphenylamine from aniline, aniline was used as the raw material, the reaction temperature was 310°C, the reaction pressure was 3MPa, the hourly volumetric space velocity of aniline was 0.2h⁻¹, the concentration of the product was analyzed by liquid chromatograph, and the conversion rate and selectivity were obtained by calculation. The results were shown in Table 3, wherein:

The time for the first increase in reaction temperature during operation (h) denoted the total operation time from the start of the reaction to the time when the aniline conversion rate decreased to 20 mol%;

Aniline conversion rate (mol%) denoted an average aniline molar conversion rate during the time for the first increase in reaction temperature during operation, 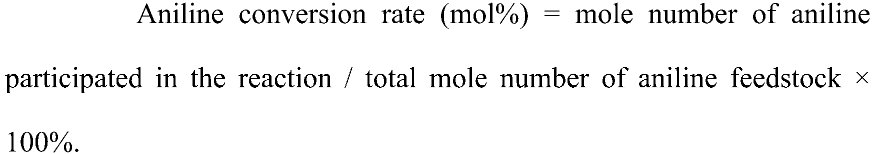 Aniline conversion rate (mol%) = mole number of aniline participated in the reaction / total mole number of aniline feedstock × 100%.

Diphenylamine selectivity (mol%) denoted an average diphenylamine molar selectivity during the time for the first increase in reaction temperature during operation, 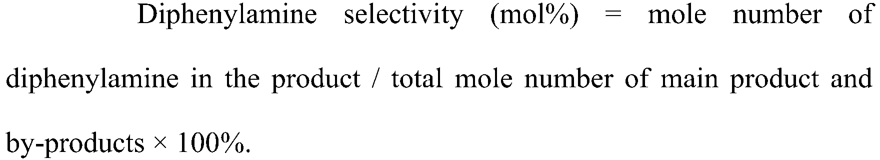 Diphenylamine selectivity (mol%) = mole number of diphenylamine in the product / total mole number of main product and by-products × 100%.

**Table 3**

| Catalyst No. | The time for the first increase in reaction temperature during operation, h | Aniline conversion rate, mol% | Diphenylamine selectivity, mol% |
|---|---|---|---|
| CAT-1 | 876 | 21.76 | 98.32 |
| CAT-2 | 652 | 21.24 | 98.35 |
| CAT-3 | 751 | 21.12 | 98.46 |
| CAT-4 | 614 | 21.20 | 98.19 |
| CAT-5 | 607 | 21.15 | 98.24 |
| CAT-6 | 544 | 21.36 | 97.53 |
| CAT-7 | 716 | 21.35 | 97.98 |
| DCAT-1 | 221 | 22.08 | 96.24 |
| DCAT-2 | 235 | 21.12 | 97.17 |
| DCAT-3 | 194 | 21.09 | 96.35 |
| DCAT-4 | 126 | 21.21 | 96.64 |
| DCAT-5 | 177 | 20.35 | 97.12 |

As can be seen from the results in Table 3, the catalysts further prepared from the modified molecular sieves obtained in the present invention have a higher diphenylamine selectivity and a longer one-way operation period.

The above content describes in detail the preferred embodiments of the present invention, but the present invention is not limited thereto. A variety of simple modifications can be made in regard to the technical solutions of the present invention within the scope of the technical concept of the present invention, including a combination of individual technical features in any other suitable manner, such simple modifications and combinations thereof shall also be regarded as the content disclosed by the present invention, each of them falls into the protection scope of the present invention.

## Claims

1. A modified molecular sieve, is **characterized in that** the modified molecular sieve comprises a β molecular sieve and an auxiliary component, and the auxiliary component is selected from at least one alkali metal;
wherein the indicative constant k of the modified molecular sieve is not less than 100;
the indicative constant k is calculated based on formula (1): $k = Q / \left(1.4909\times {a}^{- 0.558}-c\right)$ formula (1)
wherein Q denotes the standard meta-xylene adsorption capacity per 100g of modified molecular sieve, in units of g; a denotes the molar ratio of silica to alumina in the modified molecular sieve; c denotes the molar number of the auxiliary component calculated in terms of oxide per 100g of modified molecular sieve.

2. The modified molecular sieve according to claim 1, wherein the indicative constant k of the modified molecular sieve is within the range of 100-550, preferably within the range of 101-500;
preferably, the standard meta-xylene adsorption capacity per 100g of modified molecular sieve is within the range of 17-33g, more preferably within the range of 18-31g.

3. The modified molecular sieve according to claim 1 or 2, wherein the auxiliary component is selected from sodium and/or potassium;
preferably, the molar number of the auxiliary component calculated in terms of oxide per 100g of modified molecular sieve is within the range of 0.01-0.12mol, more preferably within the range of 0.02-0.1mol.

4. The modified molecular sieve according to any one of claims 1-3, wherein the molar ratio of silica to alumina in the modified molecular sieve is between 20 and 250, preferably between 25 and 200.

5. A preparation method for the modified molecular sieve, is **characterized in that** the preparation method comprises the following steps:
(1) preparing an impregnation liquid comprising an organic salt of the auxiliary component and an organic solvent;
the auxiliary component is selected from at least one alkali metal;
(2) impregnating a β molecular sieve with the impregnation liquid;
(3) purging and/or rinsing the product obtained from step (2) with an inactive fluid, such that the organic salt of the auxiliary component carries out the reaction to form a large-molecule product containing the auxiliary component, and the large-molecule product containing the auxiliary component has a molecular diameter greater than the pore diameter of the β molecular sieve to obtain a modified molecular sieve precursor;
(4) calcining the modified molecular sieve precursor.

6. The preparation method according to claim 5, wherein in step (3), purging and/or rinsing the product obtained from step (2) with an inactive fluid, such that the organic salt of the auxiliary component carries out a self-polymerization reaction to form a large-molecule product containing the auxiliary component; or
the organic salt of the auxiliary component carries out an addition reaction with the organic solvent to form a large-molecule product containing the auxiliary component.

7. A preparation method for the modified molecular sieve, is **characterized in that** the preparation method comprises the following steps:
(1) preparing an impregnation liquid comprising an organic salt of the auxiliary component and an organic solvent;
wherein the auxiliary component is selected from at least one alkali metal; the organic solvent is a substituted or unsubstituted C6-C10 cyclic hydrocarbon compound;
the molecule of said organic salt of the auxiliary component comprises a ring structure, and unsaturated functional groups capable of carrying out the addition reaction with the cyclic hydrocarbon compound;
(2) impregnating a β molecular sieve with the impregnation liquid;
(3) purging and/or rinsing the product obtained from step (2) with an inactive fluid at a temperature not lower than 80°C, and then drying to obtain a modified molecular sieve precursor;
(4) calcining the modified molecular sieve precursor.

8. The preparation method according to claim 7, wherein the ring structure is at least one selected from the group consisting of a saturated or unsaturated five-membered ring and a six-membered ring; preferably, the saturated or unsaturated five-membered ring and six-membered ring are all composed of carbon atoms;
preferably, the unsaturated functional groups are selected from carbon-carbon double bond and/or carbon-carbon triple bond.

9. The preparation method according to any one of claims 5-8, wherein the molecule of the organic salt of the auxiliary component comprises at least one unsaturated six-membered ring, preferably a benzene ring;
preferably, the organic salt of the auxiliary component has a molecular weight not more than 250g/mol, more preferably within the range of 50-220g/mol;
preferably, the organic solvent is at least one selected from the group consisting of benzene, cyclohexane, toluene, methylcyclopentane and cyclohexene.

10. The preparation method according to any one of claims 5-9, wherein the organic salt of the auxiliary component has a structure represented by formula (i); 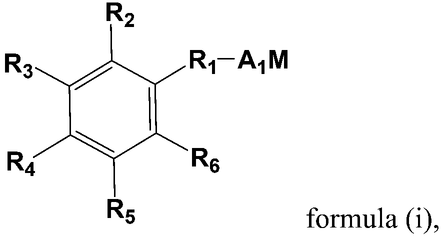 wherein R₁ is C2-C4 alkenylene or alkynylene; R₂, R₃, R₄, R₅, R₆ are each independently at least one selected from the group consisting of hydrogen atom and C1-C3 hydrocarbyl; A₁ is selected from -COO- or -SO₃-; and M is selected from at least one alkali metal ion.

11. The preparation method according to claim 10, wherein R₁ is at least one of ethenylene, propenylene and butenylene;
and/or R₂, R₃, R₄, R₅, R₆ are each independently at least one selected from the group consisting of hydrogen atom and C1-C3 hydrocarbyl;
and/or A₁ is -COO-;
and/or M is selected from K⁺ or Na⁺.

12. The preparation method according to any one of claims 5-11, wherein the organic salt of the auxiliary component is contained in an amount of 0.1-10wt%, preferably 0.5-9wt%, and the organic solvent is contained in an amount of 90-99.5wt%, preferably 90-99.5wt%, based on the total amount of the impregnation liquid;
preferably, the dosage of said impregnation liquid is 4-10mL, more preferably 5-8mL, relative to 1g of said Hβ molecular sieve.
Preferably, the impregnation time is within the range of 1-10h, more preferably within the range of 1-5h.

13. The preparation method according to any one of claims 5-12, wherein the content of said cyclic hydrocarbon compound in the inactive fluid at an outlet is not higher than 1 µg/L at the end of purging and/or rinsing process;
preferably, said inactive fluid is selected from a gas or a liquid, more preferably at least one selected from the group consisting of nitrogen, argon, helium and neon;
preferably, the purging and/or rinsing conditions comprise: the inactive fluid temperature is within the range of 80-300°C, more preferably within the range of 85-280°C, the hourly volumetric space velocity is within the range of 100-3,000h⁻¹, more preferably within the range of 200-2,000h⁻¹, the purging and/or rinsing time is within the range of 2-24h, more preferably within the range of 3-20h.

14. The preparation method according to any one of claims 5-13, wherein the calcining conditions comprise: a temperature within the range of 300-800°C, preferably within the range of 400-700°C, and a calcining time within the range of 2-24h, preferably within the range of 3-8h.

15. A modified molecular sieve produced with the preparation method according to any one of claims 5-14.

16. A catalyst, is **characterized in that** the catalyst comprises the modified molecular sieve according to any one of claims 1-4 and 15 and a binder.

17. The catalyst according to claim 16, wherein the binder is at least one selected from the group consisting of alumina, bentonite, spinel and silica;
preferably, the modified molecular sieve is contained in an amount of 55-85wt%, more preferably 58-83wt%, and the binder is contained in an amount of 15-45wt%, more preferably 17-42wt%, based on the total amount of the catalyst;
preferably, the catalyst further comprises a second auxiliary component, which is a metallic component and/or a non-metallic component;
preferably, the metal component is at least one metal element selected from group IA, group IIA, group IIIB, group IVB, group VIII, group IB, group IIB, preferably at least one of Li, La, Ce, Mg, Ca, Ba, Cu, Zn, Zr, Fe;
preferably, the non-metallic component is at least one selected from group IIIA nonmetal element, group IVA nonmetal element and group VA nonmetallic element, more preferably at least one of Si, P, B and C;
preferably, the content of said second auxiliary component calculated in terms of oxide is within the range of 0.1-5wt%, based on the total amount of the catalyst.

18. The catalyst according to claim 16 or 17, wherein the catalyst has a specific surface area within the range of 250-650 m²/g, preferably within the range of 300-600 m²/g;
preferably, the catalyst has a specific pore volume within the range of 0.2-0.55 mL/g, more preferably within the range of 0.25-0.5 mL/g.

19. The use of the modified molecular sieve according to any one of claims 1-4 and 15 or the catalyst according to any one of claims 16-18 in an aniline condensation reaction.

20. A method for synthesizing diphenylamine from aniline, is **characterized in that** the method comprises: contacting the aniline with a catalyst under the condensation reaction conditions;
wherein the catalyst is said catalyst according to any one of claims 16-18.

21. The method according to claim 20, wherein the condensation reaction conditions comprise: a reaction pressure within the range of 0.1-4MPa, preferably within the range of 0.15-3.5MPa; a reaction temperature within the range of 280-360°C, preferably within the range of 290-350°C; an aniline hourly volumetric space velocity within the range of 0.1-0.3h⁻¹, preferably within the range of 0.15-0.25h⁻¹.
